(19) Europäisches Patentamt
European Patent Office
Office européen des brevets

(11) **EP 4 760 396 A1**

(12) **EUROPEAN PATENT APPLICATION**

(43) Date of publication:
**17.06.2026 Bulletin 2026/25**

(21) Application number: **24219140.1**

(22) Date of filing: **11.12.2024**

(51) International Patent Classification (IPC):
**G03F 7/20** (2006.01)    **G01N 33/00** (2006.01)

(52) Cooperative Patent Classification (CPC):
**G03F 7/70916; G01N 33/0006; G03F 7/70516; G03F 7/7085**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ASML Netherlands B.V.**
**5500 AH Veldhoven (NL)**

(72) Inventors:
- **HULVA, Jan**
  **5500 AH Veldhoven (NL)**
- **VAN DEN BRINK, Jeroen, Aalt**
  **5500 AH Veldhoven (NL)**

(74) Representative: **ASML Netherlands B.V.**
**Corporate Intellectual Property**
**P.O. Box 324**
**5500 AH Veldhoven (NL)**

Remarks:
Claim 16 is deemed to be abandoned due to non-payment of the claims fee (Rule 45(3) EPC).

(54) **GAS SENSOR CALIBRATION SYSTEM AND METHOD**

(57)     A gas sensor calibration system, comprising:
an environment;
a gas sensor, wherein the gas sensor is configured to measure a first signal of a first gas when the environment is filled with the first gas, and a second signal of a second gas when the environment is filled with the second gas;
a pressure gauge, configured to measure a first pressure within the environment when the environment is filled with the first gas, and to measure a second pressure within the environment when the environment is filled with the second gas;
a data processor, configured to calculate a sensitivity correction factor for the second gas based on the first signal, second signal, first pressure and second pressure.

**FIG. 2**

**Description**

FIELD

The present invention relates to a gas sensor calibration system and a gas sensor calibration method for use in combination with a lithographic system.

BACKGROUND

A lithographic apparatus is a machine constructed to apply a desired pattern onto a substrate. A lithographic apparatus can be used, for example, in the manufacture of integrated circuits (ICs). A lithographic apparatus may, for example, project a pattern at a patterning device (e.g., a mask) onto a layer of radiation-sensitive material (resist) provided on a substrate.

To project a pattern on a substrate a lithographic apparatus may use electromagnetic radiation. The wavelength of this radiation determines the minimum size of features which can be formed on the substrate. A lithographic apparatus, which uses extreme ultraviolet (EUV) radiation, having a wavelength within the range 4-20 nm, for example 6.7 nm or 13.5 nm, may be used to form smaller features on a substrate than a lithographic apparatus which uses, for example, radiation with a wavelength of 193 nm.

A lithographic apparatus may use EUV radiation. EUV radiation may be produced using a plasma. A radiation system for producing EUV radiation may include a laser for exciting a fuel to provide the plasma, and a source collector module for containing the plasma. The plasma may be created, for example, by directing a laser beam at a fuel, such as droplets of a suitable material (e.g., tin), or a stream of a suitable gas or vapor, such as Xe gas or Li vapor. The resulting plasma emits output radiation, e.g., EUV radiation, which is collected using a radiation collector. The radiation collector may be a mirrored normal incidence radiation collector, which receives the radiation and focuses the radiation into a beam. The source collector module may include an enclosing structure or chamber arranged to provide a vacuum environment to support the plasma. Such a radiation system is typically termed a laser produced plasma (LPP) source.

Once the EUV radiation has been generated, it is directed through the lithographic apparatus by a plurality of optical components, such as mirrors, to a patterning surface of the patterning device, which imparts the desired pattern to the EUV radiation. Other components, such as sensors and detectors, may also be present in the lithographic apparatus. Surface degradation of the optical components can undesirably impair the properties of these components, for example reducing the reflectivity of the mirrors for the EUV radiation, thus reducing the transmission of EUV radiation. Surface degradation of other components can undesirably damage these components, for example reducing the performance and/or the operational efficiency of the component. Thus, surface degradation of certain components of the lithographic apparatus should be avoided or mitigated.

Surface degradation may be caused by, for example, the chemical reaction of contaminants with the component surface. In an EUV vacuum tool, such as an EUV lithographic apparatus and/or an EUV source, a very high level of cleanliness needs to be maintained. In particular, any contaminant gasses within an EUV vacuum tool may damage optical components within the EUV vacuum tool. The tolerable level of contaminant gasses in an EUV vacuum tool is lower than in most other vacuum tool applications.

Accurate monitoring of relevant contaminants is crucial for maintaining conditions that ensure required lifetime of contamination sensitive components, e.g. EUV mirrors. In addition, monitoring the gas composition within a lithographic system, which comprises for example a lithographic apparatus and a radiation source, allows for better process control and diagnostics of, for example, process gases, possible leakages and/or contaminants.

One method of monitoring the gas composition within the lithographic system, and thus the presence of contaminants within the lithographic system, involves using a gas sensor, or gas analysis tool. However, to ensure the correct function of the gas sensor, it needs to be calibrated after manufacturing, and the calibration needs to be verified and adjusted during its lifetime. Therefore it is desirable to provide a system and method of accurately calibrating gas sensors that monitor the gas composition within the lithographic system.

Known systems and methods of providing an accurate calibration of gas sensors may be time-consuming, involve considerable costs and increased complexity of gas supply systems. For example, using a pressurized gas bottle of a known gas mixture for calibration of the gas sensor may be difficult to implement in different locations due to regulations for transportation of pressurized gas bottles, and also due to different local regulations within the establishment in which the calibration is to be performed. Moreover, the composition of gases of the bottle can vary over time, which can impact the accuracy of the calibration.

There is a general need to improve the determination and monitoring of contaminant gasses within vacuum tools, such as an EUV vacuum tool. It may be desirable to provide a new lithographic system, and associated methods for operation, that at least partially addresses one or more problems associated with prior art arrangements, whether identified herein or otherwise.

SUMMARY

According to an aspect of the invention, there is provided a gas sensor calibration system, comprising:

an environment;
a gas sensor, wherein the gas sensor is configured to measure a first signal of a first gas when the environment is filled with the first gas, and a second signal of a second gas when the environment is filled with the second gas;
a pressure gauge, configured to measure a first pressure within the environment when the environment is filled with the first gas, and to measure a second pressure within the environment when the environment is filled with the second gas;
a data processor, configured to calculate a sensitivity correction factor for the second gas based on the first signal, second signal, first pressure and second pressure.

According to another aspect of the present invention, there is provided a method for calibrating a gas sensor in an environment, the method comprising:

measuring a first signal of a first gas when the environment is filled with the first gas, and measuring a second signal of a second gas when the environment is filled with the second gas;
measuring a first pressure within the environment when the environment is filled with the first gas, and measuring a second pressure when the environment is filled with the second gas;
calculating a sensitivity correction factor for the second gas based on the first signal, second signal, first pressure and second pressure.

BRIEF DESCRIPTION OF THE DRAWINGS

Embodiments of the invention will now be described, by way of example only, with reference to the accompanying schematic drawings, in which:

- Figure 1 is a schematic illustration of a lithographic system comprising a lithographic apparatus and a radiation source according to the invention;
- Figure 2 is a schematic illustration of a gas sensor calibration system according to the invention.
- Figure 3 depicts a flowchart of the method according to the invention.
- Figure 4 is a schematic illustration of an example of the spectrum obtained by a gas sensor.

DETAILED DESCRIPTION

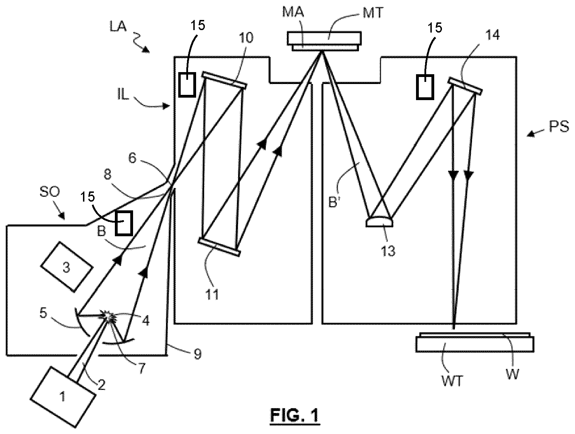

Figure 1 shows a lithographic system comprising a radiation source SO and a lithographic apparatus LA. The radiation source SO is configured to generate an EUV radiation beam B and to supply the EUV radiation beam B to the lithographic apparatus LA. The lithographic apparatus LA comprises an illumination system IL, a support structure MT configured to support a patterning device MA (e.g., a mask), a projection system PS and a substrate table WT configured to support a substrate W.

The illumination system IL is configured to condition the EUV radiation beam B before the EUV radiation beam B is incident upon the patterning device MA. Thereto, the illumination system IL may include a facetted field mirror device 10 and a facetted pupil mirror device 11. The faceted field mirror device 10 and faceted pupil mirror device 11 together provide the EUV radiation beam B with a desired cross-sectional shape and a desired intensity distribution. The illumination system IL may include other mirrors or devices in addition to, or instead of, the faceted field mirror device 10 and faceted pupil mirror device 11.

After being thus conditioned, the EUV radiation beam B interacts with the patterning device MA. As a result of this interaction, a patterned EUV radiation beam B' is generated. The projection system PS is configured to project the patterned EUV radiation beam B' onto the substrate W. For that purpose, the projection system PS may comprise a plurality of mirrors 13,14 which are configured to project the patterned EUV radiation beam B' onto the substrate W held by the substrate table WT. The projection system PS may apply a reduction factor to the patterned EUV radiation beam B', thus forming an image with features that are smaller than corresponding features on the patterning device MA. For example, a reduction factor of 4 or 8 may be applied. Although the projection system PS is illustrated as having only two mirrors 13,14 in Figure 1, the projection system PS may include a different number of mirrors (e.g. six or eight mirrors).

The substrate W may include previously formed patterns. Where this is the case, the lithographic apparatus LA aligns the image, formed by the patterned EUV radiation beam B', with a pattern previously formed on the substrate W.

A relative vacuum, i.e. a small amount of gas (e.g. hydrogen) at a pressure well below atmospheric pressure, may be provided in the radiation source SO, in the illumination system IL, and/or in the projection system PS.

The lithographic apparatus LA and radiation source SO described herein can be used in a semiconductor device manufacturing method. This method comprises receiving a substrate with a photoresist layer. The method further comprises directing (EUV) radiation from radiation source to the photoresist layer to transfer a pattern from a mask onto the photoresist layer and removing a portion of the photoresist layer to form the pattern over the substrate.

The radiation source SO shown in Figure 1 is, for example, of a type which may be referred to as a laser produced plasma (LPP) source. A laser system 1, which may, for example, include a $CO_2$ laser, is arranged to deposit energy via a laser beam 2 into a fuel (i.e., a target material), such as tin (Sn) which is provided from, e.g., a fuel supply system 3. Although tin is referred to in the following description, any suitable fuel may be used. The fuel may, for example, be in liquid form, and may, for example, be a metal or alloy. The fuel supply system 3 may comprise a nozzle configured to direct the fuel, e.g. in the form of droplets, along a trajectory towards a source plasma formation region 4. The laser beam 2 is incident upon the fuel at the source plasma formation region 4. The deposition of laser energy into the tin creates a source plasma 7 at the source plasma formation region 4. Radiation, including EUV radiation, is emitted from the source plasma 7 during de-excitation and recombination of electrons with ions of the source plasma 7.

The EUV radiation from the source plasma 7 is collected and focused by a reflective surface 5. According to embodiments the reflective surface is a collector. The collector comprises, for example, a near-normal incidence radiation collector 5 (sometimes referred to more generally as a normal-incidence radiation collector). The collector 5 may have a multilayer mirror structure which is arranged to reflect EUV radiation (e.g., EUV radiation having a desired wavelength such as 13.5 nm). The collector 5 may have an ellipsoidal configuration, having two focal points. A first one of the focal points may be at the source plasma formation region 4, and a second one of the focal points may be at an intermediate focus 6, as discussed below.

The laser system 1 may be spatially separated from the radiation source SO. Where this is the case, the laser beam 2 may be passed from the laser system 1 to the radiation source SO with the aid of a beam delivery system (not shown) comprising, for example, suitable directing mirrors and/or a beam expander, and/or other optics. The laser system 1, the radiation source SO and the beam delivery system may together be considered to be a radiation system.

Radiation that is reflected by the collector 5 forms the EUV radiation beam B. The EUV radiation beam B is focused at intermediate focus 6 to form an image at the intermediate focus 6 of the source plasma present at the source plasma formation region 4. The image at the intermediate focus 6 acts as a virtual radiation source for the illumination system IL. The radiation source SO is arranged such that the intermediate focus 6 is located at or near to an opening 8 in an enclosing structure 9 of the radiation source SO.

Although Figure 1 depicts the radiation source SO as a laser produced plasma (LPP) source, any suitable source such as a free electron laser (FEL) or a discharge produced plasma (DPP) source may be used to generate EUV radiation.

An EUV vacuum tool may comprise a plurality of vacuum environments. The performance of a vacuum tool is dependent on how clean each vacuum environment within the vacuum tool is. This is particularly important for an EUV vacuum tool, such as an EUV lithographic system, in which very clean vacuum environments are required over a large dynamic range. Any contaminant gasses within a vacuum environment may severely damage components in the vacuum environment, such as optical components. A contaminant gas as referred to herein may be any gas that, when present in a quantity higher than a tolerance level for the gas, may potentially be detrimental to any aspect of the performance of a vacuum tool, such as an EUV vacuum tool. The levels and/or types of gasses present in each of the vacuum environments therefore needs to be closely monitored. The availability of the EUV vacuum tool is dependent on the state of the vacuum environments because the EUV beam should not be switched on if the levels of any gasses and/or contaminants present are not within the operational specification (i.e. below maximum tolerable levels). When the levels of any gasses present are not within the operational specification, the vacuum environments of the EUV vacuum tool need to be reconditioned in order to prevent excessive damage to the optical components, or other components, occurring.

The gas types, and levels, within a vacuum environment of an EUV vacuum tool may be monitored by a gas sensor. Each vacuum environment within the EUV vacuum tool may comprise a gas sensor, so that the EUV vacuum tool comprises a plurality of gas sensors. A single vacuum environment within an EUV vacuum tool may comprise a plurality of gas sensors. For example, if a vacuum environment within an EUV vacuum tool comprises more than one set of optical components, a separate gas sensor may be positioned close to each set of optical components.

As shown in Figure 1, a gas sensor 15 may be used to determine and/or monitor contaminant gasses within the radiation source SO and/or the lithographic apparatus LA. The gas sensor may be used to determine and/or monitor contaminant gases within, for instance, the illumination system IL, and/or the projection system PS.

The gas sensor 15 may be placed within an environment of the vacuum tool, or in connection to an environment of the vacuum tool. The gas sensor may comprise, for example, a sample tube in fluid communication with the inside of an environment such as the vacuum chamber of a vacuum tool, such as a EUV vacuum tool. There may be a pressure sensor, or pressure gauge, for measuring the pressure within the vacuum chamber.

The use of one or more gas sensors in an EUV vacuum tool allows the detection, measurement and monitoring of the gases within the EUV vacuum tool. Accurate detection, measurement and/or monitoring of relevant process gases and contaminants such as H2, N2, H2O, O2 and/or hydrocarbons, is crucial for maintaining conditions that ensure required lifetime of contamination sensitive components, e.g. EUV mirrors. In response to the gas detection, measurement and/or monitoring, reconditioning operations, and/or any other appropriate actions, may be performed if contaminant gasses are detected and/or the measured levels contaminant gases are higher than an tolerable level.

The gas sensor may be, for example, a measurement system that might accomplish quantitative measurements of a gas, such as a residual gas analyser (RGA). An RGA is a mass spectrometer for process control and contamination monitoring of a vacuum environment. RGAs may be used to determine if an EUV vacuum tool is ready for exposure (i.e. the EUV beam may be switched on), monitor the levels of contaminant gasses within the EUV vacuum tool and detect any vacuum leaks.

A known implementation of an RGA comprises an ion source, a sample chamber containing rarified gas, an ion accelerator and an ionization chamber. The ion source may include an electron generating filament that is an electron emitter. The electron generating filament may be a cathode. Electrons from the ion source are accelerated towards an anode. The electrons enter the sample chamber, through one or more openings in the walls of the sample chamber, and ionize gas molecules within the sample chamber. The ion accelerator draws the resulting ions out of the sample chamber and focusses them into a beam of ions that are injected into the ionization chamber. The ionization chamber may comprise one or more analysis modules for analyzing and/or detecting the ions. The RGA may, for example, generate and output a spectrum showing the masses and concentrations of the detected ions. An RGA therefore allows the detection, measurement and monitoring of gases within a vacuum environment.

It may be difficult to determine if a gas sensor is operating correctly or if the gas sensor is faulty. The consequences of incorrect operation of a gas sensor may include, for example, reduced availability of the EUV vacuum tool and the optical components of the EUV vacuum tool being damaged by contaminant gases. The gas sensor may be faulty due to wrong calibration, or deviations from the latest calibration, as gas sensors calibrations are known for drifting over time.

To ensure a correct function of the gas sensor, the gas sensor needs to be calibrated after manufacturing, and the calibration needs to be verified and adjusted during the lifetime of the gas sensor. Calibration of the gas sensor is used to determine the sensitivity correction factors for individual gas species. In an EUV vacuum tool, the vacuum atmosphere may be, for example, composed of hydrogen as a dominant gas, and also of contaminant species such as N2, O2, H2O.

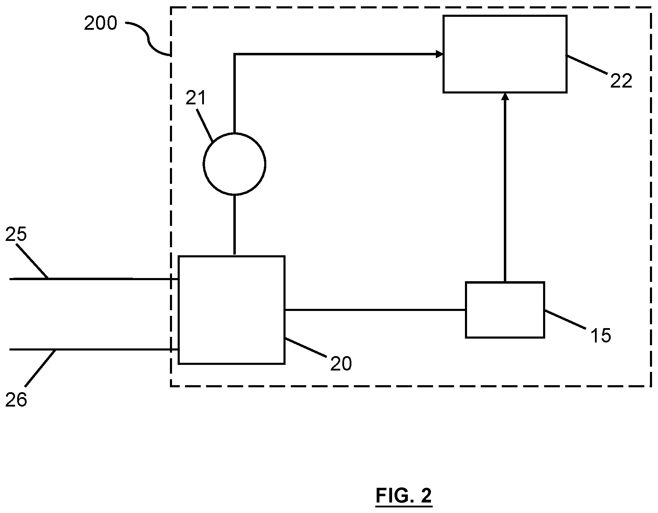

Figure 2 shows a schematic illustration of a gas sensor calibration system 200 according to the invention. A first gas source 25 may provide a first gas to an environment 20 of the gas sensor calibration system 200. The first gas may be, for example, hydrogen gas. A pressure sensor 21, or gas independent pressure sensor 21, is configured to measure the total pressure within the environment 20 in which it is placed in or connected to. The pressure sensor 21 may preferably be gas independent. A gas sensor 15 may be able measure a first signal, which relates to the amount of the first gas within the environment 20. When the environment 20 is, for example, substantially filled with the first gas at a given pressure, the pressure sensor 21 is able to measure the total pressure within the environment 20, which corresponds to the pressure of the first gas. The sensitivity of the gas sensor 15 to the first gas may be calculated by using the gas sensor signal measurement of the first gas, and the pressure sensor measurement when the first gas is the dominant species within the environment 20. The gas species may be an atom, ion or molecule that constitutes a gas. A sensitivity of the gas sensor 15 to the first gas may be calculated with the use of a data processor 22. The data processor 22 is configured to use the measurements of the pressure sensor 21 and the gas sensor 15 in order to calculate the sensitivity of the gas sensor 15 to a given gas X, according to the equation below:

$$S_X = \frac{I_X}{P_X} \qquad\qquad (\text{Eq. 1})$$

Where $S_X$ is the sensitivity of the gas sensor 15 to a given gas X, $I_X$ is the measured gas sensor signal of gas X, and $P_X$ is the total pressure within the environment 20 when a dominant species within the environment 20 is gas X. Gas X may be the dominant species within the environment 20 when, for example, the volume of gas X is between 95% and 100% of the total volume of the gas present in the environment 20. Gas X may be the dominant species within the environment 20 when, for example, the volume of gas X is between 99% and 100% of the total volume of the gas present in the environment 20. The range of volume of gas X as the total volume of the gas present in the environment 20 will impact the accuracy of the measurement, and consequently the calibration. Thus for a more accurate calibration it is preferred that the volume of gas X is more than 99% of the total volume of gas present in the environment.

Similarly to the described above, the sensitivity of the gas sensor 15 to different gases may be obtained. A second gas source 26 may provide a second gas to the environment 20. The second gas may be, for example, nitrogen gas, or argon gas. When the environment 20 is, for example, filled with the second gas at a given pressure, the pressure sensor 21 is able to measure the total pressure within the environment 20, which corresponds to the pressure of the second gas. A gas sensor 15 may be able measure a second signal, which relates to the amount of the second gas within the environment 20. The sensitivity of the gas sensor 15 to the second gas may be calculated by using the gas sensor signal measurement of the second gas, and the pressure sensor measurement when the second gas is the dominant species within the environment 20. The sensitivity of the gas sensor 15 to the second gas may be calculated with the use of the data processor 22. The data processor 22 is configured to use the measurements of the pressure sensor 21 and the gas sensor 15 in order to calculate the sensitivity of the gas sensor 15 to a given gas X, according to Eq.1.

For the case when a given gas, such as gas Z, is not the dominant gas species within the environment 20, the partial pressure of gas Z ($pp_Z$) within the environment 20 is calculated as the ratio between the measured gas sensor signal of gas Z ($I_Z$) and the sensitivity of the gas sensor 15 to gas Z ($S_Z$):

$$pp_Z = \frac{I_Z}{S_Z} \qquad \text{(Eq. 2)}$$

The sensitivity $S_Z$ of the gas sensor 15 to a given gas Z may also be expressed in terms of the sensitivity $S_X$ of the gas sensor 15 to a gas X and a sensitivity correction factor $A_Z$:

$$S_Z = \frac{S_X}{A_Z} \qquad \text{(Eq. 3)}$$

Eq. 3 assumes that the sensitivity for the non-dominant gas may be expressed as being proportional to the sensitivity of another gas. Eq. 3 may be re-written as follows:

$$A_Z = \frac{S_X}{S_Z} \qquad \text{(Eq. 4)}$$

In a lithographic system, such as an EUV vacuum tool, the sensitivity of the gas sensor to different gases may be measured as shown in Eq. 1. In case such measurement is performed for two (or more) different gases, for example for a gas X and a gas Z, then the sensitivity correction factor $A_Z$ may be calculated from Eq.4.

For example, Eq. 1 may be used to measure the sensitivity of the gas sensor to a first gas, such as hydrogen gas, and to a second gas, such as nitrogen gas. Using the measured sensitivity of the gas sensor to the first and second gases, it is possible to retrieve the sensitivity correction factor for the sensitivity of the gas sensor to nitrogen gas with respect to the sensitivity of the gas sensor to hydrogen gas.

Using Eq. 1, then Eq. 4 may be re-written as follows:

$$A_{Second\ gas} = \frac{I_{First\ gas}}{P_{First\ gas}} \cdot \frac{P_{Second\ gas}}{I_{Second\ gas}} \qquad \text{(Eq. 5)}$$

Where:

$I_{First\ gas}$ is the first signal of the first gas, the first signal relating to an amount of the first gas in the environment, $P_{First\ gas}$ is the first pressure, as measured when the first gas is the dominant species within the environment, $I_{second\ gas}$ is the second signal of the second gas, the second signal relating to an amount of the second gas in the environment, and $P_{Second\ gas}$ is the second pressure as measured when the second gas is the dominant species within the environment.

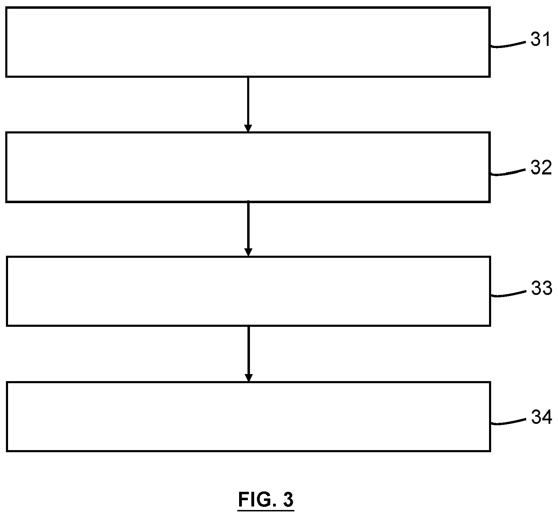

A flowchart of the method according to the invention is shown in Figure 3. Figure 3 shows a method for calibrating a gas sensor in an environment, the method comprising: measuring a first signal of a first gas when the environment is filled with the first gas, and measuring a second signal of a second gas when the environment is filled with the second gas (32); measuring a first pressure within the environment when the environment is filled with the first gas, and measuring a second pressure when the environment is filled with the second gas (33); calculating a sensitivity correction factor for the second gas based the first signal, second signal, first pressure and second pressure (34).

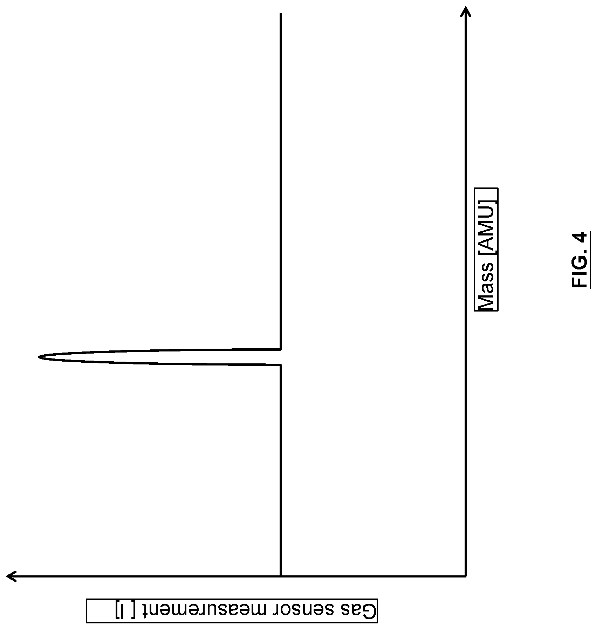

The gas sensor signal as measured for a gas species may be, for example, a spectrum of the gas species, such as the spectrum shown in Figure 4. The spectrum of a gas species may consist of, for example, the gas sensor measurement as a function of the atomic mass unit (AMU). For example, if the gas sensor is an RGA, the full spectra of calibrated species (e.g. nitrogen) may contain gas molecule clusters (for nitrogen N3, N4) that are formed due to interaction between molecules during ionization at higher pressures. The cluster-related peaks can be used to track performance of the RGA for low intensity signals. According to the invention, such calibration system and method can be used to calibrate any gas species provided it is possible to supply enough of the calibrated species to measure the absolute pressure of the species by a pressure gauge. The sensitivity correction factors obtained by the calibration system and method described may also be benchmarked against other known calibration methods to ensure accuracy and continuity in partial pressure interpretation.

Thus, the sensitivity correction factor may be used to calibrate the gas sensor for any measurable gases.

From Eq. 2 and Eq. 3, it is possible to obtain the partial pressure of a gas Z from the measured gas sensor signal $I_Z$ of gas Z, the sensitivity $S_X$ of the gas sensor 15 to a gas X and the sensitivity correction factor $A_Z$

$$pp_Z = I_Z \cdot \frac{A_Z}{S_X} \qquad\qquad (\text{Eq. } 6)$$

The gas sensor calibration system and method according to the invention may measure sensitivity factors for multiple dominant species.

According to the invention, the gas sensor may be calibrated online in the system in which it operates. The gas sensor calibration system and method described herein may be implemented to lithographic systems, such as such as an EUV vacuum tool, using spectra that are already measured during normal operation of the EUV vacuum tools. Due to no need of additional hardware, the gas sensor calibration system and method described herein has significant advantage over currently developed methods for gas sensor calibration. Additionally, the gas sensor calibration system and method described herein does not have strict requirements on gas cleanliness.

The gas sensor calibration system and method described herein may also be used in combination with other calibration system or methods in order to calibrate the gas sensor for a variety of gas species. For example, the gas sensor calibration system and method described herein may be used in combination with offline measurements and/or calibrations of the gas sensor.

Although specific reference may be made in this text to the use of lithographic apparatus in the manufacture of ICs, it should be understood that the lithographic apparatus described herein may have other applications. Possible other applications include the manufacture of integrated optical systems, guidance and detection patterns for magnetic domain memories, flat-panel displays, liquid-crystal displays (LCDs), thin-film magnetic heads, etc.

Although specific reference may be made in this text to embodiments of the invention in the context of a lithographic apparatus, embodiments of the invention may be used in other apparatus. Embodiments of the invention may form part of a mask inspection apparatus, a metrology apparatus, or any apparatus that measures or processes an object such as a wafer (or other substrate) or mask (or other patterning device). These apparatus may be generally referred to as lithographic tools. Such a lithographic tool may use vacuum conditions or ambient (non-vacuum) conditions.

Although specific reference may have been made above to the use of embodiments of the invention in the context of optical lithography, it will be appreciated that the invention, where the context allows, is not limited to optical lithography and may be used in other applications, for example imprint lithography.

Where the context allows, embodiments of the invention may be implemented in hardware, firmware, software, or any combination thereof. Embodiments of the invention may also be implemented as instructions stored on a machine-readable medium, which may be read and executed by one or more processors. A machine-readable medium may include any mechanism for storing or transmitting information in a form readable by a machine (e.g., a computing device). For example, a machine-readable medium may include read only memory (ROM); random access memory (RAM); magnetic storage media; optical storage media; flash memory devices; electrical, optical, acoustical or other forms of propagated signals (e.g. carrier waves, infrared signals, digital signals, etc.), and others. Further, firmware, software, routines, instructions may be described herein as performing certain actions. However, it should be appreciated that such descriptions are merely for convenience and that such actions in fact result from computing devices, processors, controllers, or other devices executing the firmware, software, routines, instructions, etc. and in doing that may cause actuators or other devices to interact with the physical world.

While specific embodiments of the invention have been described above, it will be appreciated that the invention may be practiced otherwise than as described. The descriptions above are intended to be illustrative, not limiting. Thus it will be apparent to one skilled in the art that modifications may be made to the invention as described without departing from the scope of the claims set out below.

**Claims**

1. A gas sensor calibration system, comprising:

    an environment;
    a gas sensor, wherein the gas sensor is configured to measure a first signal of a first gas when the environment is filled with the first gas, and a second signal of a second gas when the environment is filled with the second gas;
    a pressure gauge, configured to measure a first pressure within the environment when the environment is filled with the first gas, and to measure a second pressure within the environment when the environment is filled with the second gas;
a data processor, configured to calculate a sensitivity correction factor for the second gas based on the first signal, second signal, first pressure and second pressure.

2. The gas sensor calibration system of claim 1, wherein the gas sensor is configured for use in a lithographic system, and/or an EUV vacuum tool.

3. The gas sensor calibration system according to claim 2, wherein the environment is a vacuum environment of the lithographic system, and/or the EUV vacuum tool.

4. The gas sensor calibration system of any previous claim, wherein the gas sensor is a residual gas analyser (RGA).

5. The gas sensor calibration system of any previous claim, wherein the first gas is hydrogen gas, nitrogen gas, or argon gas.

6. The gas sensor calibration system of any previous claim, wherein the second gas is hydrogen gas, nitrogen gas, or argon gas.

7. The gas sensor calibration system of any previous claim, wherein the gas sensor calibration system may be further used in combination with an offline measurement.

8. The gas sensor calibration system of any previous claim, wherein the sensitivity correction factor for the second gas is calculated according to the following equation:

$$A_{Second\ gas} = \frac{I_{First\ gas}}{P_{First\ gas}} \cdot \frac{P_{Second\ gas}}{I_{Second\ gas}}$$

where $I_{First gas}$ is the first signal of the first gas, $P_{First gas}$ is the first pressure, $I_{Second gas}$ is the second signal of the second gas, and $P_{Second\ gas}$ is the second pressure.

9. A lithographic system comprising a gas sensor calibration system according to any of the preceding claims.

10. The lithographic system of claim 9, wherein the environment is an EUV radiation source, and/or a EUV lithographic apparatus.

11. A method for calibrating a gas sensor in an environment, the method comprising:

measuring a first signal of a first gas when the environment is filled with the first gas, and measuring a second signal of a second gas when the environment is filled with the second gas;
measuring a first pressure within the environment when the environment is filled with the first gas, and measuring a second pressure when the environment is filled with the second gas;
calculating a sensitivity correction factor for the second gas based on the first signal, second signal, first pressure and second pressure.

12. The method of claim 11, wherein the gas sensor is used in a lithographic system, and/or a EUV vacuum tool.

13. The method of claim 12, wherein the environment is a vacuum environment of the lithographic system, and/or the vacuum tool.

14. The method of any of claims 11-13, wherein the gas sensor is a residual gas analyser (RGA).

15. The method of any of claims 11-14, wherein the first gas is hydrogen gas, nitrogen gas, or argon gas.

16. The method of any of claims 10-15, wherein the second gas is hydrogen gas, nitrogen gas, or argon gas.

FIG. 1

FIG. 2

**FIG. 3**

Mass [AMU]

Gas sensor measurement [ I ]

FIG. 4

Europäisches
Patentamt

European
Patent Office

Office européen
des brevets

## EUROPEAN SEARCH REPORT

Application Number

EP 24 21 9140

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MALYSHEV O ET AL: "In situ ultrahigh vacuum residual gas analyzer "calibration"", JOURNAL OF VACUUM SCIENCE AND TECHNOLOGY A, AMERICAN INSTITUTE OF PHYSICS, 2 HUNTINGTON QUADRANGLE, MELVILLE, NY 11747, vol. 26, no. 6, 30 October 2008 (2008-10-30), pages 1474-1479, XP012114043, ISSN: 0734-2101, DOI: 10.1116/1.2990856 * the whole document * | 1-15 | INV. G03F7/20 G01N33/00 |
| A | DE 10 2020 209482 A1 (ZEISS CARL SMT GMBH [DE]) 3 February 2022 (2022-02-03) * the whole document * | 1-15 | |
| A | "Operating Manual and Programming Reference, Models RGA100, RGA200, and RGA300 Residual Gas Analyzer, Revision 1.8", STANFORD RESEARCH SYSTEMS , May 2009 (2009-05), XP055613161, Retrieved from the Internet: URL:https://www.thinksrs.com/downloads/pdfs/manuals/RGAm.pdf [retrieved on 2019-08-15] * Chapter 7 "RGA Tuning" * | 1-15 | |
| | | | TECHNICAL FIELDS SEARCHED (IPC) |
| | | | G03F G01N |

The present search report has been drawn up for all claims

| Place of search | Date of completion of the search | Examiner |
|---|---|---|
| Munich | 29 July 2025 | Maslankiewicz, Pawel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☒ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

## ANNEX TO THE EUROPEAN SEARCH REPORT
## ON EUROPEAN PATENT APPLICATION NO.

EP 24 21 9140

This annex lists the patent family members relating to the patent documents cited in  the above-mentioned European search report.
The members are as contained in the European Patent Office EDP file on
The European Patent Office is in no way liable for these particulars which are merely given for the purpose of information.

29-07-2025

| Patent document cited in search report | Publication date | Patent family member(s) | Publication date |
|---|---|---|---|
| DE 102020209482 A1 | 03-02-2022 | DE 102020209482 A1 | 03-02-2022 |
| | | WO 2022023312 A1 | 03-02-2022 |

EPO FORM P0459

For more details about this annex : see Official Journal of the European Patent Office, No. 12/82